(19) Europäisches Patentamt
European Patent Office
Office européen des brevets

(11) Veröffentlichungsnummer: **0 470 462 A2**

(12) **EUROPÄISCHE PATENTANMELDUNG**

(21) Anmeldenummer: **91112635.7**

(22) Anmeldetag: **27.07.91**

(51) Int. Cl.5: **C07C 45/40**, C07C 49/167, C07C 49/16

(30) Priorität: **09.08.90 DE 4025188**

(43) Veröffentlichungstag der Anmeldung: **12.02.92 Patentblatt 92/07**

(84) Benannte Vertragsstaaten: **DE FR GB**

(71) Anmelder: **BAYER AG**
**W-5090 Leverkusen 1 Bayerwerk(DE)**

(72) Erfinder: **Baasner, Bernd, Dr.**
**Wagnerstrasse 83**
**W-5060 Bergisch Gladbach 2(DE)**
Erfinder: **Lorentzen, Jens, Dr.**
**An der Ruthen 2**
**W-5000 Köln 80(DE)**

(54) **Verfahren zur Herstellung von Halogenmethylketonen, insbesondere von 1,1,1-Trifluoraceton.**

(57) Halogenmethylketone werden aus Nitroverbindungen hergestellt, in dem man diese in alkoholischer Lösung mit Natrium- oder Kaliumalkoholatlösung versetzt, auf eine Temperatur im Bereich -10 bis -100 °C abkühlt und dann das Gemisch bei -10 bis -100 °C mit Ozon reagieren läßt. Das Verfahren eignet sich insbesondere zur Herstellung von 1,1,1-Trifluoraceton aus 1,1,1-Trifluor-2-nitro-propan.

EP 0 470 462 A2

Die vorliegende Erfindung betrifft ein besonders vorteilhaftes Verfahren zur Herstellung von Halogenmethylketonen. Halogenmethylketone, insbesondere 1,1,1-Trifluoraceton, sind wichtige Zwischenprodukte zur Herstellung von biologisch aktiven Verbindungen (siehe z.B. J. Med. Chem. 1975 (18), 895, J. Med. Chem. 1983 (26), 950 und Bull. Soc. Chim. France 1986, 933).

Es sind schon Verfahren zur Herstellung von Halogenmethylketonen, insbesondere von 1,1,1-Trifluoraceton, bekanntgeworden. So kann man ausgehend von Trifluoressigsäuremethylester durch Kondensation mit Essigester und anschließender säurekatalysierter Spaltung des als Zwischenprodukt gebildeten Trifluoracetessigsäureethylesters Trifluoraceton herstellen. Durch Selbstkondensation des Essigesters entstehen hohe Anteile an Nebenprodukten (Ausbeute an gewünschtem Produkt nur 54 %) und es werden darüber hinaus lange Reaktionszeiten benötigt. (siehe Bull. class. Sci. Acad. roy. Belg. [5]12, 692 (1926); Bull. Acad. Belg. [5]13, 175; J. Am. Chem. Soc. 69, 1819 (1947); J. Am. Chem. Soc. 74, 1426 (1952)).

Bei der Behandlung von Trihalogenessigsäuren mit Alkylmagnesiumbromiden in Dibutylether werden Trihalogenmethyl-alkyl-ketone erhalten (siehe J Chem. Soc. 1956, 835). In ähnlicher Weise wird bei der Behandlung von Trifluormethylmagnesiumiodid mit Acetylchlorid oder Acetonitril in Dibutylether 1,1,1-Trifluoraceton erhalten (siehe J. Chem. Soc. 1954, 1273).

Weitere bekannte Verfahren zur Herstellung von 1,1,1-Trifluoraceton sind die mit Quecksilber(II)-salzen katalysierte Umsetzung von 3,3,3-Trifluorpropin mit wäßrigem Methanol (siehe J. Am. Chem. Soc. 74, 650 (1952)) oder wäßriger Schwefelsäure (siehe J Chem. Soc. 1952, 3483) und die Oxidation von 1,1,3,3,3-Pentafluor-2-methyl-propen mit Kaliumpermanganat in saurer wäßriger Lösung (siehe J. Chem. Soc. 1953, 3565).

Nachteilig bei diesen Verfahren ist, daß die benötigten Ausgangsprodukte nur in aufwendigen Synthesen zugänglich und deshalb sehr teuer sind (z.B. 3,3,3-Trifluorpropin), technisch schwierig handhabbare Umsetzungen durchgeführt werden müssen (z.B. Grignard-Reaktionen), ökologisch besonderen Aufwand erfordernde Hilfsstoffe benötigt werden (z.B. Quecksilbersalze) und/oder niedrige Ausbeuten ergeben.

Es wurde nun ein Verfahren zur Herstellung von Halogenmethylketonen der Formel (I) gefunden

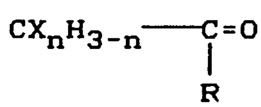

(I),

in der

X für Halogen,

n für 1, 2 oder 3 und

R für gegebenenfalls substituiertes $C_1$-$C_{20}$-Alkyl steht,

das dadurch gekennzeichnet ist, daß man eine Nitroverbindung der Formel (II)

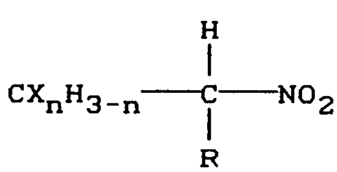

(II),

in der

X, n und R die bei Formel (I) angegebene Bedeutung haben,

in Alkohol mit Natrium- oder Kaliumalkoholatlösung versetzt, auf eine Temperatur im Bereich -10 bis -100° C abkühlt und dann das Gemisch bei -10 bis -100° C mit Ozon reagieren läßt.

Als Alkohole kommen beispielsweise Methanol, Ethanol, Isopropanol, Propanol, n-Butanol, t-Butanol, sec.-Butanol, Pentanol und/oder iso-Amylalkohol infrage.

Als Alkoholatlösung kommen Lösungen von Natrium- oder Kaliumalkoholaten in beliebigen Alkoholen infrage. Bevorzugt sind Natrium- oder Kaliumalkoholate mit 1 bis 4 C-Atomen in Verbindung mit einem der oben genannten Alkohole als Lösungsmittel. Besonders bevorzugt sind Lösungen mit Natrium- oder Kaliummethylat in Methanol.

Bei den Formeln (I) und (II) kann X beispielsweise für Fluor, Chlor und/oder Brom stehen. Vorzugsweise steht es für Fluor. n steht vorzugsweise für 2 oder 3, besonders bevorzugt für 3.

Soweit R einen substituierten Alkylrest bedeutet, kommen als Substituenten beispielsweise in Frage: Halogenatome, Arylgruppen mit 6 bis 10 C-Atomen, C = O-Gruppen, COOR'-Gruppen mit R' = $C_1$-$C_6$-Alkyl

und Acetalgruppen der Formel (III)

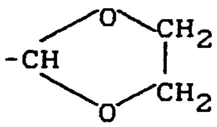

(III) .

Vorzugsweise steht R für gegebenenfalls mit Fluor, Chlor, Brom, Phenyl, substituiertem Phenyl, Naphthyl und/oder substituiertem Naphthyl, substituiertes $C_1$-$C_6$-Alkyl. Besonders bevorzugt steht R für Methyl, Ethyl, Propyl, Butyl oder Pentyl, ganz besonders bevorzugt für Methyl. Das bedeutet, ganz besonders bevorzugt wird erfindungsgemäß aus 1,1,1-Trifluor-2-nitro-propan 1,1,1-Trifluoraceton hergestellt.

Die als Ausgangsmaterialien benötigten Verbindungen der Formel (II) sind beispielsweise gemäß EP-A 116 885 und gemäß EP-A 116 886 oder auf analoge Weise dazu erhältlich.

Man kann das erfindungsgemäße Verfahren so durchführen, daß man zunächst die Verbindung der Formel (II) in möglichst wasserfreiem Alkohol löst und dann Natrium- oder Kaliumalkoholat in fester Form oder gelöst in einem Alkohol zugibt. Natrium- oder Kaliumalkoholat kann man beispielsweise in Mengen von 0,8 bis 1,2 Mol pro Mol Verbindung der Formel (II) einsetzen. Bevorzugt sind 0,9 bis 1,1 Mol Natrium- oder Kaliumalkoholat, insbesondere 1 Mol Natriummethylat, jeweils bezogen auf 1 Mol der Verbindung der Formel (II).

Die Zugabe des Alkoholates kann beispielsweise bei Temperaturen im Bereich -80 bis +60 °C, vorzugsweise bei -20 bis +40 °C erfolgen.

Vor der Zugabe des Ozons wird das Reaktionsgemisch auf -10 bis -100 °C, vorzugsweise -50 bis -78 °C abgekühlt und dann bei einer Temperatur in diesen Bereichen mit Ozon umgesetzt.

Ozon kann man beispielsweise gasförmig, z.B. im Gemisch mit Sauerstoff in das Reaktionsgemisch einleiten, oder in gelöster Form, z.B. gelöst in einem der oben aufgeführten Alkohole, zufügen. Um einen vollständigen Umsatz sicherzustellen ist es vorteilhaft, Ozon in mindestens äquimolarer Menge, bezogen auf die Verbindung der Formel (II), einzusetzen. Beispielsweise kann man bezogen auf die Verbindung der Formel (II) 1 bis 1,2 Mole Ozon einsetzen, wobei man einen geringen Überschuß an Ozon an einer bestehenbleibenden Blaufärbung des Reaktionsgemisches erkennen kann.

Das Reaktionsgemisch kann man dann aufarbeiten, indem man es sich auf Raumtemperatur erwärmen läßt und eventuell noch vorhandenes überschüssiges Ozon vor, während und/oder nach dem Erwärmen auf Raumtemperatur ausbläst, z.B. mit Stickstoff oder einem anderen inerten Gas. In dem dann verbleibenden Gemisch liegt das gebildete Halogenmethylketon der Formel (I) im allgemeinen als Hydrat, Acetal und/oder Halbacetal vor. Für viele weitere Umsetzungen kann man das so hergestellte Halogenmethylketon der Formel (I) in Form des nach dem Erwärmen auf Raumtemperatur und gegebenenfalls Ausblasen von überschüssigem Ozon verbleibenden Gemisches einsetzen.

Man kann solche Gemische auch zur Isolierung des gebildeten Halogenmethylketons der Formel (I) aufarbeiten. Dabei kann man, z.B. gegebenenfalls nach Zusatz von Dimethylsulfid, leicht-flüchtige Bestandteile, insbesondere Methanol, abdampfen, den Rückstand mit Ether aufnehmen, die etherische Lösung mit Wasser und/oder Kochsalzlösung waschen, anschließend trocknen, z.B. mit Natriumsulfat, dann Einengen und durch Destillation und/oder Kristallisation das hergestellte Halogenmethylketon der Formel (I) isolieren.

Im Falle der erfindungsgemäßen Herstellung niedrigsiedender, leicht flüchtiger Halogenmethylketone, z.B. von 1,1,1-Trifluoraceton mit einem Siedepunkt von 20 bis 22 °C, das zudem unter den Reaktionsbedingungen noch leicht das Hydrat oder das Halbacetal mit dem jeweiligen Alkohol bildet, können nach der Umsetzung und Entfernung eventuell noch vorhandenen Ozons durch Zugabe von Phosphorpentoxid und anschließender Destillation in reiner Form oder als alkoholische Lösung, die in der Regel für nachfolgende Reaktionen eingesetzt werden kann, isoliert werden. Für einige Folgereaktionen ist es am zweckmäßigsten, direkt die Ozon-freie Rohlösung nach einer Bestimmung des Gehaltes an Halogenmethylketon einzusetzen. Die Ausbeuten an Halogenmethylketonen bei der erfindungsgemäßen Herstellung betragen im allgemeinen zwischen 70 und 95 %, bezogen auf die eingesetzte Nitroverbindung der Formel (II). Die Reinheiten der isolierten Halogenmethylketone liegen dabei im allgemeinen zwischen 82 und 98 % (gaschromatografisch bestimmt).

Es ist ausgesprochen überraschend, daß auf die erfindungsgemäße Weise Halogenmethylketone der Formel (I) so gut hergestellt werden können, denn es ist bekannt, daß z.B. aus 1,1,1-Trifluor-2-nitro-propan in einem basischen Milieu eine Fluorid-Eliminierung stattfindet (siehe US-A 4 840 969).

<u>Beispiele</u>

Beispiel 1

Eine Lösung von 10,1 g (70 mmol) 1,1,1-Trifluor-2-nitro-propan in 100 ml absolutem Methanol wurde mit 3,87 g (7,2 mol) festem Natriummethanolat versetzt und auf -78 ° C gekühlt. Anschließend wurden bei derselben Temperatur 900 ml einer gesättigten methanolischen Ozonlösung zugefügt und 1,5 Stunden bei -78 ° C belassen. Überschüssiges Ozon wurde dann mittels eines Stickstoffstroms bei -78 ° C ausgeblasen. In dieser Rohlösung lag nach spektroskopischer Untersuchung (IR, Raman-, $^1$H-NMR- und $^{19}$F-NMR-Spektroskopie) das 1,1,1-Trifluoraceton, sein Hydrat und das Halbacetal mit Methanol vor.

Nach Zugabe von Phosphorpentoxid wurde anschließend bei Normaldruck das Produkt in Methanol bei einer Temperatur bis 66 ° C abdestilliert. Das so erhaltene Gemisch kann ohne weitere Isolierung des Trifluoracetons für weitere Umsetzungen eingesetzt werden.

Zur weiteren Identifikation zur Bestimmung der Ausbeute wurde der oben beschriebene Ansatz wiederholt und die nach der Destillation erhaltene Lösung in eine Lösung von 29,7 g (150 mmol) 2,4-Dinitrophenylhydrazin in 400 ml 20 gew.-%iger wässriger Schwefelsäure bei 20 ° C unter Rühren eingetropft (analog J. Am. Chem. Soc. 69, 1819 (1947)). Das ausgefallene 2,4-Dinitrophenylhydrazon des 1,1,1-Trifluoracetons wurde abgesaugt, mit Wasser nachgewaschen und getrocknet. Es wurden 17,1 g des Hydrazons mit einem Schmelzpunkt von 137 - 138 ° C erhalten. Das entspricht einer Ausbeute von 83 %, bezogen auf eingesetztes 1,1,1-Trifluor-2-nitropropan.

Beispiel 2

Eine Lösung von 12,3 g (70 mmol) 1,1-Dichlor-1-fluor-2-nitropropan in 100 ml absolutem Methanol wurde mit 3,87 g (7,2 mol) festem Natriummethanolat versetzt und auf -78 ° C gekühlt. Anschließend wurden bei derselben Temperatur 900 ml einer gesättigten methanolischen Ozonlösung zugefügt und 1,5 Stunden bei -78 ° C belassen. Überschüssiges Ozon wurde dann mittels eines Stickstoffstroms bei -78 ° C ausgeblasen. In dieser Rohlösung lag nach spektroskopischer Untersuchung (IR, Raman-, $^1$H-NMR- und $^{19}$F-NMR-Spektroskopie) das 1,1-Dichlor-1-fluoraceton, sein Hydrat und das Halbacetal mit Methanol vor.

Zur weiteren Identifikation und zur Bestimmung der Ausbeute wurde der oben beschriebene Ansatz wiederholt und die Rohlösung in eine Lösung von 29,7 g (150 mmol) 2,4-Dinitrophenylhydrazon in 400 ml 20 gew.-%iger wäßriger Schwefelsäure bei 20 ° C unter Rühren eingetropft. Das ausgefallene 2,4-Dinitrophenylhydrazon des 1,1-Dichlor-1-fluoracetons wurde abgesaugt, mit Wasser nachgewaschen und getrocknet. Es wurden 10,7 g des Hydrazons mit einem Schmelzpunkt von 145-146 ° C erhalten. Das entspricht einer Ausbeute von 83 %, bezogen auf eingesetztes 1,1-Dichlor-1-fluor-2-nitropropan.

**Patentansprüche**

**1.** Verfahren zur Herstellung von Halogenmethylketonen der Formel (I)

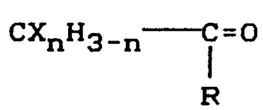 (I),

in der

X für Halogen,

n für 1, 2 oder 3 und

R für gegebenenfalls substituiertes $C_1$-$C_{20}$-Alkyl steht,

dadurch gekennzeichnet, daß man eine Nitroverbindung der Formel (II)

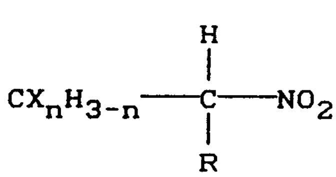 (II),

in der

X, n und R die bei Formel (I) angegebene Bedeutung haben,

in Alkoholen mit Natrium- oder Kaliumalkoholatlösung versetzt, auf eine Temperatur im Bereich -10 bis -100 °C abkühlt und dann das Gemisch bei -10 bis -100 °C mit Ozon reagieren läßt.

**2.** Verfahren nach Anspruch 1, dadurch gekennzeichnet, daß in den Formel (I) und (II)

X für Fluor, Chlor und/oder Brom,

n für 2 oder 3, und

R für einen gegebenenfalls mit Halogenatomen, Arylgruppen mit 6 bis 10 C-Atomen, C=O-Gruppen, COOR'-Gruppen mit R' = $C_1$-$C_6$-Alkyl und/oder Acetalgruppen der Formel (III)

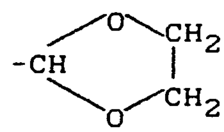 (III)

substituierten $C_1$-$C_{20}$-Alkylrest steht.

**3.** Verfahren nach Ansprüchen 1 und 2, dadurch gekennzeichnet, daß man als Verbindung der Formel (II) 1,1,1-Trifluor-2-nitro-propan einsetzt und als Halogenmethylketon der Formel (I) 1,1,1-Trifluoraceton erhält.

**4.** Verfahren nach Ansprüchen 1 bis 3, dadurch gekennzeichnet, daß man 0,8 bis 1,2 Mol Natrium- oder Kaliumalkoholat pro Mol Nitroverbindung der Formel (II) einsetzt und das Alkoholat bei Temperaturen im Bereich -80 bis +60 °C zugibt.

**5.** Verfahren nach Ansprüchen 1 bis 4, dadurch gekennzeichnet, daß man die Umsetzung mit Ozon bei Temperaturen im Bereich -50 bis -78 °C durchführt.

**6.** Verfahren nach Ansprüchen 1 bis 5, dadurch gekennzeichnet, daß man Ozon gasförmig im Gemisch mit Sauerstoff oder gelöst in Alkohol zufügt.

**7.** Verfahren nach Ansprüchen 1 bis 6, dadurch gekennzeichnet, daß man 1 bis 1,2 Mole Ozon pro Mol der Nitroverbindung der Formel (II) einsetzt.

**8.** Verfahren nach Ansprüchen 1 bis 7, dadurch gekennzeichnet, daß man nach Beendigung der Reaktion überschüssigen Ozon ausbläst.

**9.** Verfahren nach Ansprüchen 1 bis 7, dadurch gekennzeichnet, daß man nach der Reaktion überschüssigen Ozon ausbläst, dann, gegebenenfalls nach Zusatz von Dimethylsulfid, leicht-flüchtige Bestandteile abdampft, den Rückstand mit Ether aufnimmt, die etherische Lösung mit Wasser und/oder Kochsalzlösung wäscht, anschließend trocknet, einengt und durch Destillation und/oder Kristallisation das hergestellte Halogenmethylketon der Formel (I) isoliert.